# EUROPEAN PATENT SPECIFICATION

(11) **EP 2 459 237 B1**
(45) Date of publication and mention of the grant of the patent: **21.08.2013**
(21) Application number: 10747277.1
(22) Date of filing: 14.07.2010
(51) Int. Cl.: A61L 9/03

(54) **AIR FRESEHENER**
DUFTSTOFFABGABEVORRICHTUNG
DISPOSITIF DE DISTRIBUTION D'UN PARFUM

(30) Priority: 27.07.2009 IT BS20090140
(43) Date of publication of application: 06.06.2012
(73) Proprietor: CMS Di Colosio Mauro, 25050 Zone, Brescia (IT)
(72) Inventor: COLOSIO, Mauro, I-25050 Zone, BRESCIA (IT); FRACCAROLI, Davide, I-25015 Desenzano del Garda, BRESCIA (IT)
(74) Representative: Chimini, Francesco
(86) International application number: PCT/IB2010/053216
(87) International publication number: WO 2011/013024

(56) References cited:
- WO-A1-99/03514
- WO-A1-2005/021053
- WO-A1-2006/061803
- WO-A2-2004/033020
- WO-A2-2005/123153

## Description

The present invention relates to a dispenser device of a substance suitable for distributing a substance contained therein in a controlled manner. In particular, said dispenser device is of such size and dimensions as to be considered a portable device and thereby suitable for use in numerous fields and applications.

Substances which can be distributed by the device according to the invention are in particular liquid substances, but may also be powders or gases. For example, the device according to the invention has been designed for use in the cosmetics, medical and cleaning sectors. In particular, depending on the substance used, the proposed device may act as a stain-remover, freshener, disinfectant, cleaner etc.

The distribution or dispensing of the substance occurs in a controlled manner, channelling it outwards through a porous material, the porosity being chosen depending on the density of the substance and on the application.

A number of similar devices have already been proposed. For example, a disposable stain-remover device is known of wherein the stain-removing substance is contained in a glass phial which the user breaks in the moment of need. Examples of said devices are shown in documents WO 2004/033020A2, WO 2005/021053A1, WO 2006/061803A1 and WO 99/03514A1.

However, such devices, as well as being complicated to manufacture, are not particularly safe or convenient to use inasmuch as requiring breakage of a glass container, as a result of which they have not become very widespread and in addition cannot be used in various fields of use.

The purpose of the present invention is to propose a dispenser device of a substance contained therein able to overcome the above cited drawbacks and therefore particularly convenient and simple to use and easy to adapt to use in numerous fields.

Such purpose is achieved by a device according to claim 1. The dependent claims describe preferred or advantageous embodiments of the dispenser device.

The characteristics and advantages of the device according to the invention will be apparent from the description below, made by way of non limiting examples of its preferred embodiments, wherein:

- Figures 1, 1a and 1b illustrate the dispenser device according to the invention, in a first embodiment, in an exploded perspective view, assembled in an inactive position, and during the dispensing phase of a substance, respectively;

- Figures 2, 2a and 2b show analogous views of the device to the previous, but in axial cross-section;

- Figures 3 and 3a are enlarged cross-section views of the device at the level of the perforation tip, in an inactive position and in a perforation position, respectively;

- Figure 4 is an end view of the container-body of the device;

- Figures 5, 5a and 5b show the dispenser device according to the invention, in a second embodiment, in an exploded perspective view, assembled in an inactive position, and during the dispensing phase of a substance, respectively;

- Figures 6, 6a and 6b show analogous views of the device to the previous, but in axial cross-section;

- Figures 7 and 7a are enlarged cross-section views of the device at the level of one of the closure caps of the device, in an inactive position and in a perforation position of the cap, respectively;

- Figure 7b is an enlarged perspective view of the perforation tip only of the device in the second embodiment;

- Figure 8, 8a and 8b show, in axial cross-section, different embodiments of the dispenser tip;

- Figures 9, 9a and 9b show, in axial cross-section, the dispenser device in an embodiment variation of the first embodiment, in an exploded view, assembled in an inactive position, and in the dispensing phase of a substance, respectively;

- Figure 10 is an end view of the container of the device in figure 9;

- Figure 11 is a section of the container at the level of the perforation tip; and

- Figure 12 is a table containing some indicative examples of porosity of the material releasing the substance to be dispensed, depending on the type and density of the substance.

In said drawings, reference numerals 1; 100; 200 globally denote a dispenser device of a substance according to the invention.

According to a general embodiment, the dispenser device of a substance comprises a container-body 10; 110; 210 of an elongated shape in which a tank 12; 112; 212 containing the substance to be dispensed is at least partially contained.

The container-body 10; 110; 210 extends around a longitudinal axis X and has two open extremities 14, 14'; 114; 214, 214' opposite each other. For example, the container-body 10; 110; 210 is a cylindrical shape.

Each tank 12; 112; 212 is hermetically sealed by a cap 16; 116; 216. The cap 16; 116; 216 may, for example, be screwed to the tank, coupled under pressure, or welded using ultrasound or heat-welding.

In the container-body 10; 110; 210 at least one perforation tip 18; 118; 218 is also at least partially housed, able to perforate a respective cap 16; 116; 216 to let the substance out of the tank. To such purpose, either said tank 12; 112; 212 or perforation tip 18; 118; 218 is axially movable towards the other by a manual movement so as to cause the perforation of the cap.

A dispenser tip 20; 120; 220 is joined to at least one extremity 14'; 114; 214'. Said dispenser tip 20; 120; 220 is made so as to be able to channel the substance coming out of the respective tank 12; 112; 212 in a calibrated manner.

"Calibrated" dispensing or distribution of the substance is taken to mean that, depending on the substance, its density and the intended application of the device, the structure and/or material composing the dispenser tip 20; 120; 220 can be chosen so as to calibrate the release of the substance in the desired quantities and/or speed for a specific use.

Preferably, the dispenser tip 20; 120; 220 is made from porous material.

According to one embodiment, the dispenser tip 20; 120; 220 is made from a sintered material.

According to one variation, said tip is made from spongy material.

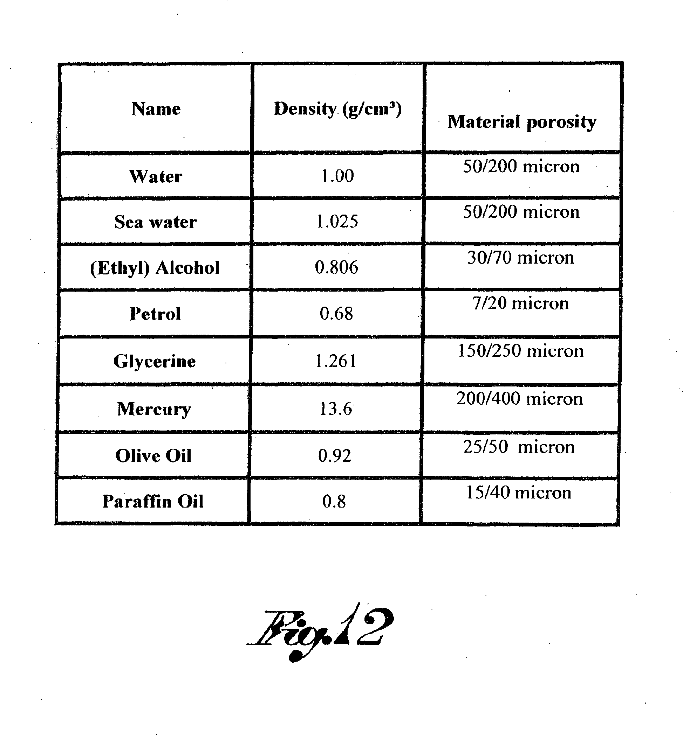

The table in figure 12 gives some examples of porosity of the material depending on the type and density of the substance to be dispensed.

In one alternative embodiment, the dispenser tip 20; 120; 220 is made from fabric.

According to a preferred embodiment, the substance coming out of the tank 12; 112; 212 flows into the dispenser tip 20; 120; 220 by gravity. In other words, once the cap 16; 116; 216 has been broken, to release the substance it is sufficient to incline the device from the horizontal position.

According to one embodiment, the perforation tip 18; 118, has at last one longitudinal groove 19; 119 able to allow transit of the substance from the tank towards the dispenser tip when said perforation tip penetrates the cap 16; 116.

According to one embodiment, the dispenser tip 20; 120; 220 comprises a shaft 20'; 120'; 220' at least partially housed with calibrated interference in a respective portion of extremity of the container-body and a head 20"; 120"; 220" projecting from said portion of extremity. In one embodiment, the head 20"; 120"; 220" has a bigger transversal section than the shaft 20'; 120'; 220' so as to abut against the rim delimiting the aperture of the container-body 10; 110; 210.

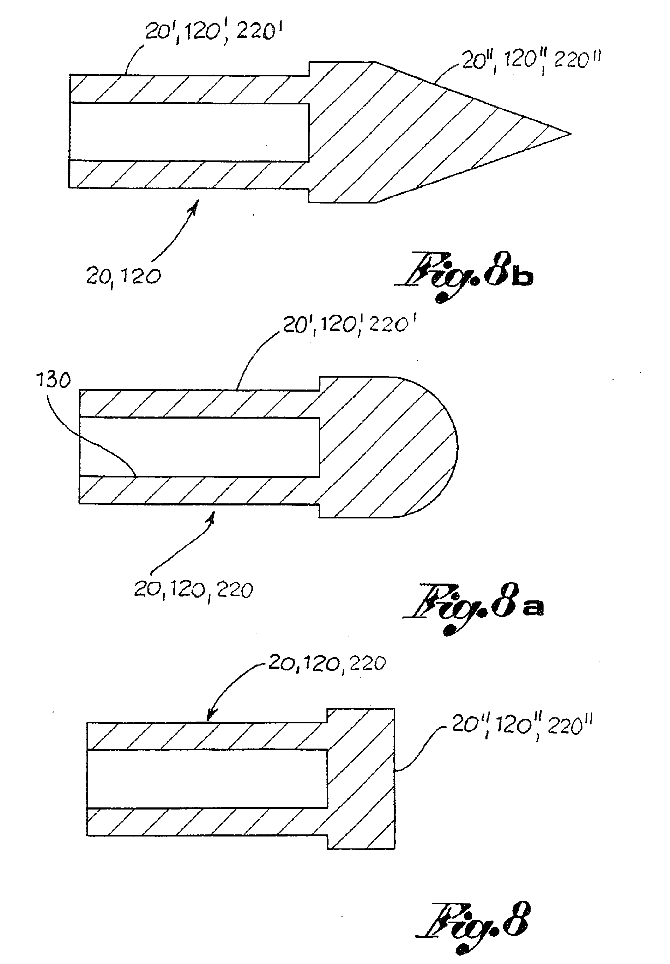

Depending on the application, the head 20"; 120"; 220" may have a flat (figure 8), convex (figure 8a), or pointed (figure 8b) dispensing surface.

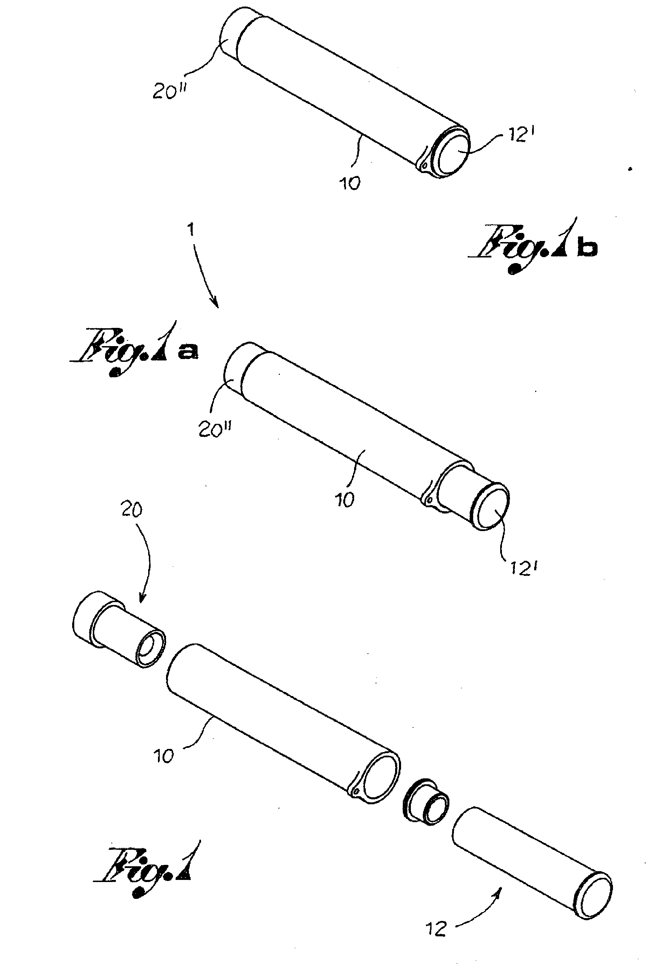

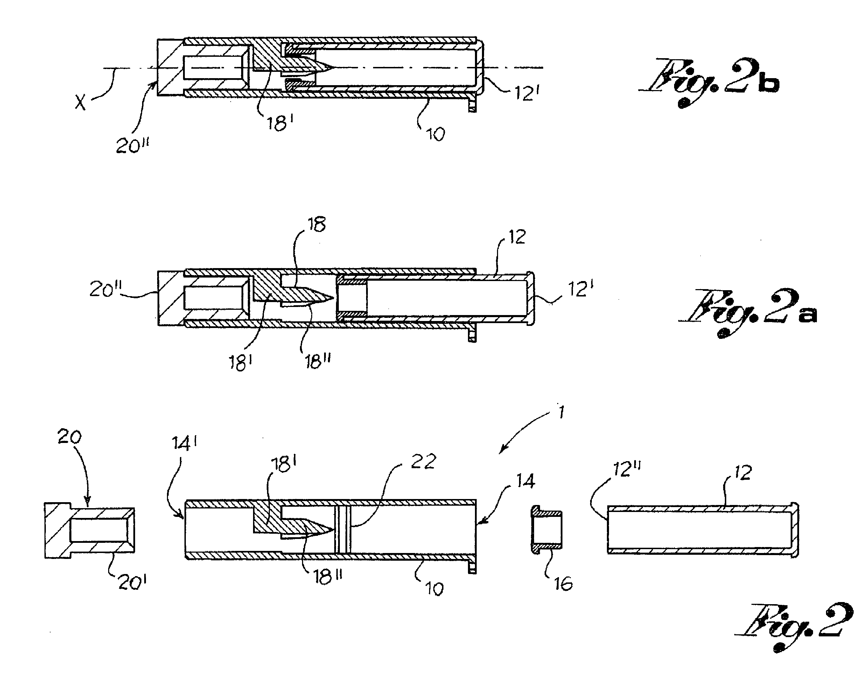

According to a first particular embodiment shown in figures 1-4, relative to a container with a single tank 12 and single dispenser tip 20, such tank 12 is in the shape of a capsule having a proximal extremity 12' projecting from one extremity 14 of the container-body 10 and a distal extremity 12" fitted with a cap 16, facing the inside of said container-body 10.

In this embodiment, the perforation tip 18 is attached to the inside of the container-body 10, while the capsule 12 is axially movable towards said tip 18, by means of manual pressure exerted on the proximal extremity 12' to cause breakage of the cap 16.

The dispenser tip 20 is joined to the extremity 14' of the container-body 10 opposite the capsule 12.

Preferably, the perforation tip 18 is made in one piece with the container-body 10, for example by moulding. More specifically, the perforation tip 18 comprises an attachment portion 18' for the connection to the container-body 10, formed for example by three radial equidistant arms, and perforation head 18", extending axially from said attachment portion 8' towards the cap 16 of the capsule 12.

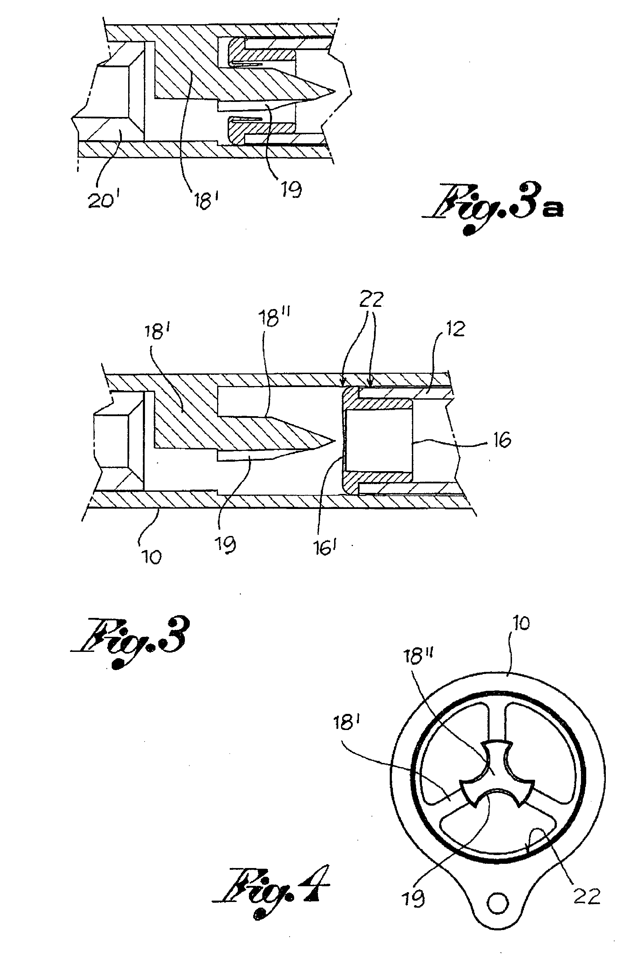

As may be seen from the end view in figure 4, three longitudinal grooves 19 are made in the perforation head 18".

Preferably, in addition, the capsule 12 is inserted with a shaped coupling in the container-body 10. In other words, the capsule 12 has an outer diameter substantially equal to the inner diameter of the container-body 10. Advantageously, in yet other terms, the capsule 12 is able to slide with calibrated interference along the inner walls of the container-body 10.

According to a preferred embodiment, in the outer lateral surface of the capsule 12 and/or in the inner surface of the container-body 10, means of transversal interference between the capsule 12 and body 10 are provided so as to define the positioning of the capsule 12 in relation to the container-body 10 when the capsule is rearward of the perforation tip 18, and having in addition the function of a seal against leakage of the substance from the capsule. For example, said means of transversal interference comprise a pair of annular bosses 22.

In the embodiment in figures 1-4, therefore, the capsule 12, which is the mobile element axially movable by means of manual pressure, is joined to one extremity 14 of the container-body 10; the dispenser tip 20 is joined to the opposite extremity. The perforation tip 18 is placed substantially in the middle of the container-body 10. In addition, the perforation head 18" of said perforation tip 18 extends along the longitudinal axis X of the container-body 10, so as to centrally perforate the front wall 16' of the cap 16. This allows the fluid contained in the tank 12 to flow evenly towards the dispenser tip 20, without the need to provide special means of guiding or conducting the fluid.

When the capsule 12 is pressed fully to break the cap 16, the proximal extremity 12' of the capsule itself aligns with the rim delimiting the aperture of the container-body 10. For example, said proximal extremity 12' defines an annular abutment shoulder against said rim, so as to act as a stop element to the stroke of the capsule 12.

Figures 3 and 3a show how the front wall 16' of the cap 16, facing the perforation tip 18, is perforated and opened by said tip, advantageously in an axial symmetrical manner when the capsule 20 is pressed fully.

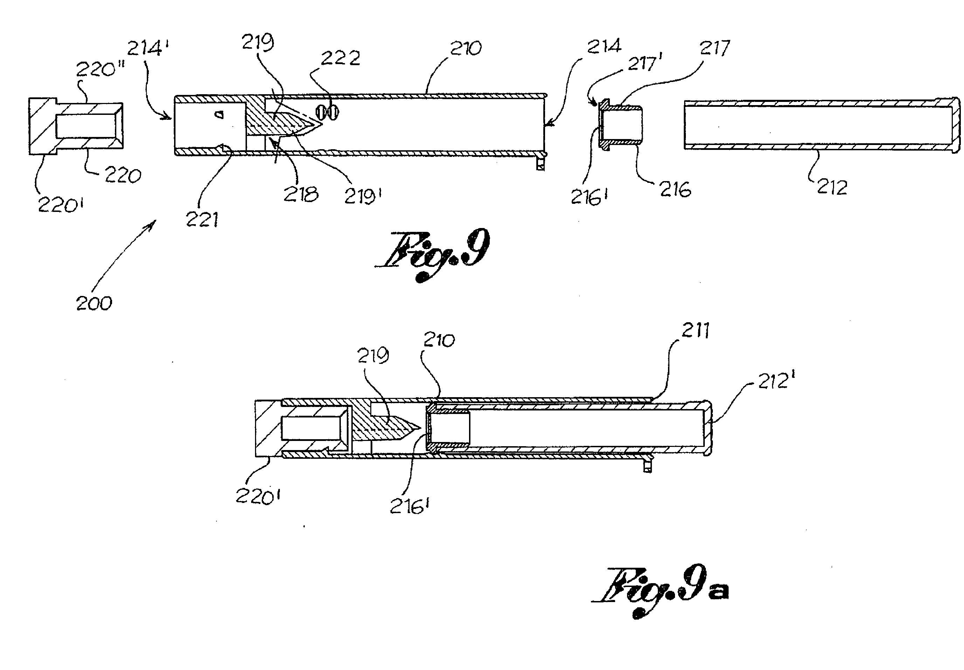

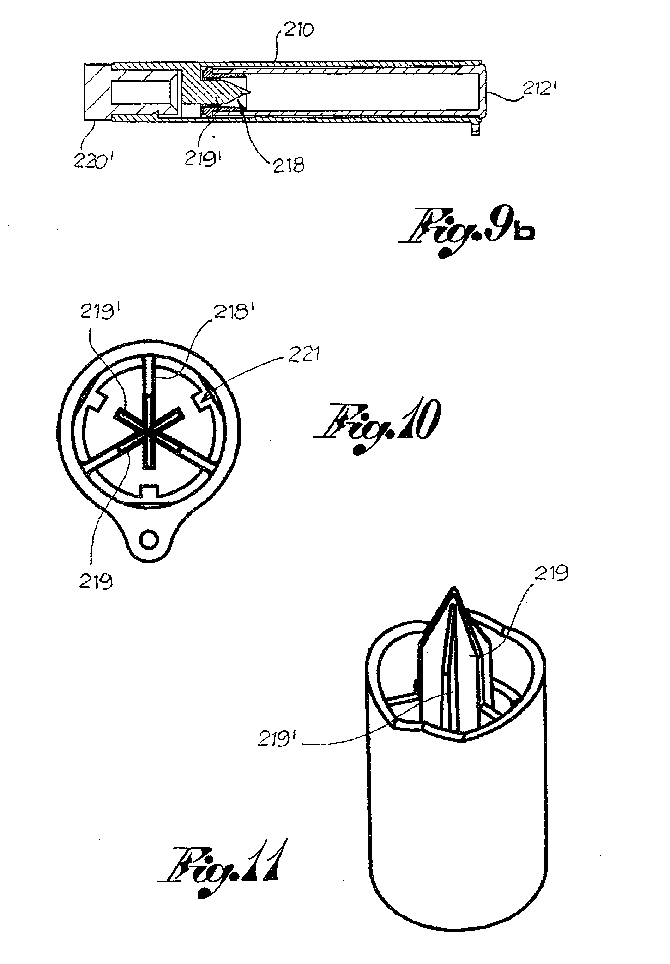

Figures 9-11 show an embodiment variation 200 of the dispenser device in figures 1-4. The dispenser device 200 has the same general structure and the same functioning principle as the device 1 illustrated in figures 1-4, but has a number of further or different features.

The perforation tip 218 again comprises an attachment portion 218' for connection to the lateral wall of the container-body 210, formed of a plurality of radial arms, preferably three, and a perforation head 218" which extends axially from said attachment portion. Said perforation head 218" comprises a plurality of primary perforation blades 219, for example three in number, radially equidistant to each other and converging axially towards the cap 216 so as to form the apex of the perforation head 218". In addition, said perforation head 218" comprises a plurality of secondary perforation blades 219', for example three in number, radially equidistant to each other and alternated with the primary perforation blades 219. Said secondary perforation blades 219' also axially converge towards the cap 216 but terminate in a position rearward of the apex of the perforation head.

Advantageously, the primary perforation blades 219 extend from the arms of the attachment portion 218', while the secondary perforation blades 219' are attached longitudinally along one side to the primary perforation blades 219.

Such structure of the perforation tip 218 facilitates the initial perforation of the wall 216' of the cap 216 thanks to the pointed apex formed by the converging extremities of the primary perforation blades 219. This makes it possible to impress a minimal thrust force on the tank 212 for the perforation of the front wall 216' of the cap 216. As soon as perforation has taken place, the secondary perforation blades 219' also intervene helping to evenly open the front wall 216' of the cap 216 making it recede into the cap and adhere to the inner wall of the same. In other words, the radial extension of the primary and secondary blades is substantially equal to the extension of the front wall 216' of the cap 216. Consequently, the fluid contained in the tank 212 is entirely free to flow from the cap 216. In addition, the blades-shape of the perforation tip 218 makes it possible to minimise contact with the fluid coming out of the cap 216. In any case, the perfectly axial symmetrical shape of the perforation tip allows the fluid to strike the dispenser tip 220 in a uniform manner.

According to a preferred embodiment of the device 200, on the outer lateral surface of the cap 216 there is at least one annular boss 217 able to form a hermetic seal with the inner lateral wall of the tank 212 so as to prevent the exit of the fluid to be dispensed. Said annular boss 217 may be made in one piece with the body of the cap or welded, for example by ultrasound, gluing or heat-welding.

In addition, the front wall 216' of the cap 216, which in one embodiment is composed of a reduced thickness membrane so as to require a minimal force for its perforation, is surrounded by a protective ledge 217' projecting frontally in relation to the plane of the front surface 216'.

According to a preferred embodiment, on the inner lateral surface of the portion of the container-body 210 holding the dispenser tip 220 there are a plurality of anchorage teeth 221 able to interact with the outer lateral surface of the shaft 220" of said dispenser tip 220 in such a way as to axially retain it. This makes it possible to avoid the use of glues or other chemical products which could cause reactions with the substances contained in the tank.

According to a preferred embodiment, the extremity 214 of the container-body facing the inside of the tank 212 is fitted with a containment rim 211, preferably rounded, able to surround the proximal extremity 212' of the tank 212 when this is fully pressed against the perforation tip 218. Such containment rim 211 makes it possible to prevent the voluntary or involuntary extraction of the tank 212.

In figures 9-9b one may also observe the radial bosses 222 made on the inner surface of the tank-body 210 to retain the tank 212, for example, by interference with the cap 216, in the inactive position, rearward from the perforation tip 218.

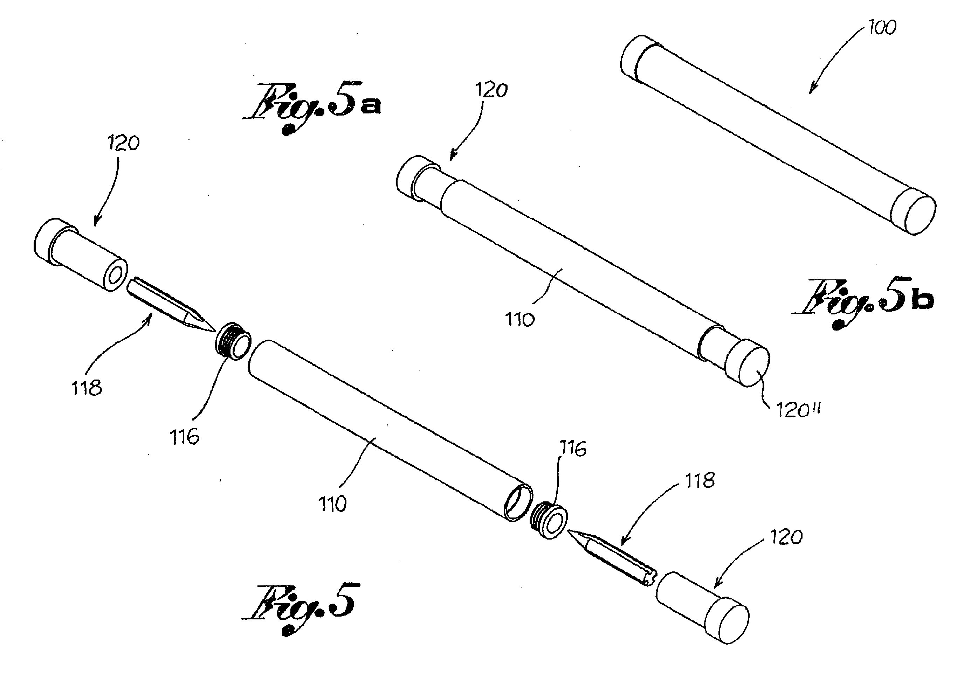

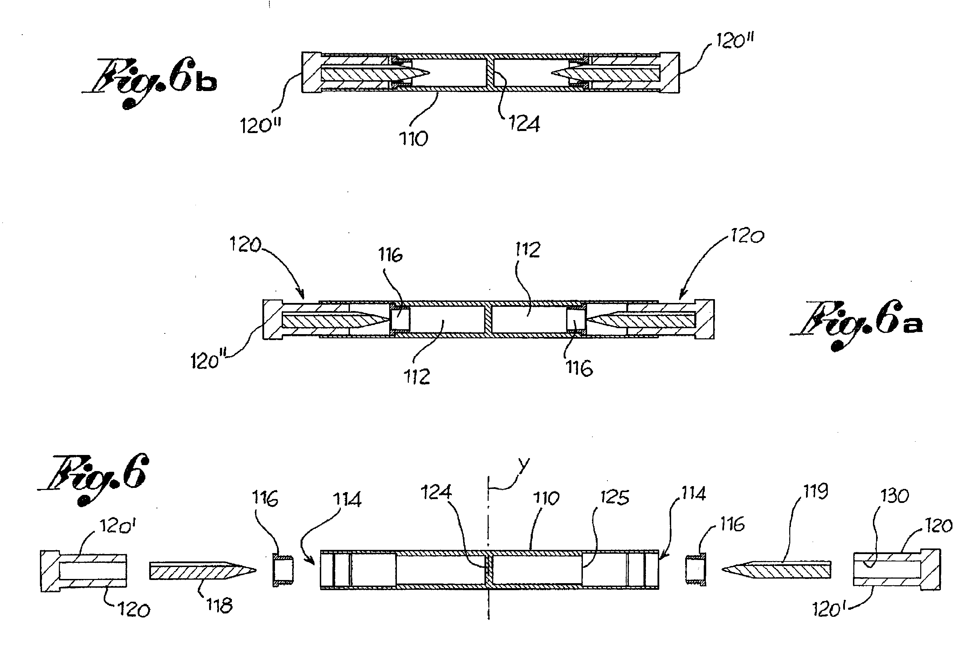

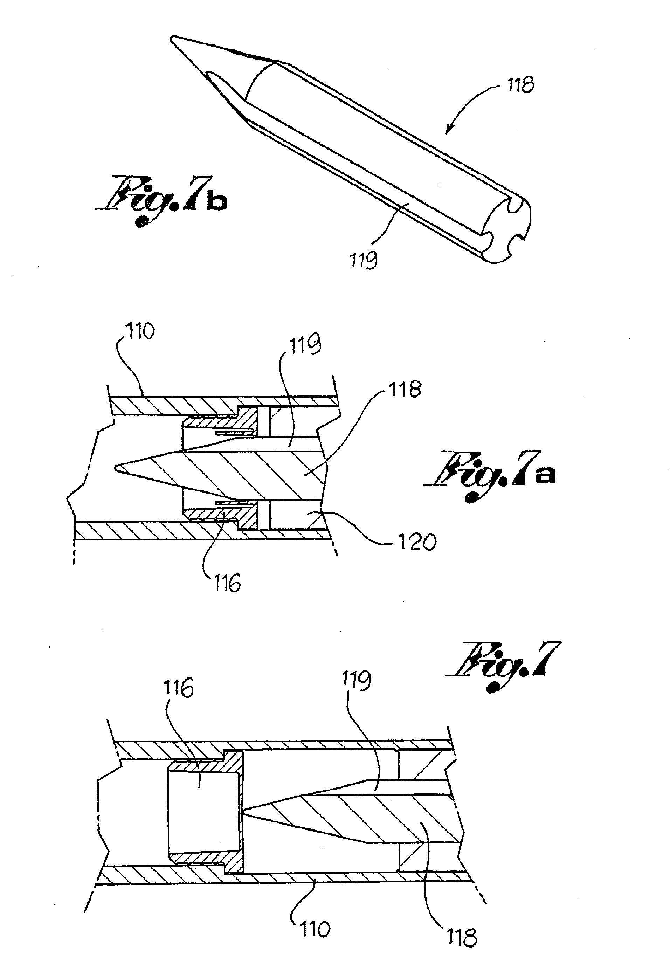

According to one embodiment variation shown in figures 5-8, at least one tank is made in an inner portion of the container-body 100, between a bottom wall 124 and the closure cap of the tank 116. Preferably, the bottom wall 124 is made in one piece with the container-body 110.

The cap 116 is attached to the container-body 110 in an inner position in relation to the extremity 114 of said container-body to which the dispenser tip 120 is joined. In this embodiment, the dispenser tip 120 is axially movable in relation to the container-body 110 by means of a manual action so as to cause the corresponding movement of the perforation tip 118 to break the cap 116. In other words, in this embodiment, the tank 112 is fixed, being preferably made directly in the container-body, and it is the perforation tip 118 which moves axially in relation to it.

According to a preferred embodiment, the perforation tip 118 receives the axial movement from the dispenser tip 120, in turn suitable for being manually pressed by the user.

According to one advantageous embodiment, the perforation tip 118 is supported by the dispenser tip. In particular, said perforation tip 118 is at least partially housed in a portion of the dispenser tip 120 facing towards the inside of the container-body 110. For example, the perforation tip 118 is press-fitted into an axial hole 130 made in the shaft 120' of the respective dispenser tip 120.

Given that the user acts manually on the dispenser tip 120, in applications which require the sterilisation of the tip, a removable protective cover may be provided.

In one embodiment, each tank 112 defines, at the extremity opposite the bottom wall 124, a shoulder 125 which the cap 116 abuts against.

In a second particular embodiment shown in figures 5-8, the device 100 comprises a double tank 112 and double dispenser tip 120. Advantageously, such device has a symmetrical structure in relation to its median transversal axis Y.

For example, the bottom wall 124 is made on the median transversal axis Y of the container-body 110 so as to define the communal bottom of two adjacent and opposite tanks 112.

It is worth noting that, in all the embodiments described above, a tank 12; 112 may be divided, for example in an axial direction, by at least one inner wall so as to be able to contain more than one dose of substance to be dispensed. Correspondingly, the perforation tip 18; 118 will be designed to perforate, following a first movement, the cap 16; 116 and, following a further subsequent axial movement, the inner wall.

The device according to the invention has, in all the embodiments described, an extremely compact and linear structure, easy to transport and hold. For example, its dimensions are similar to those of a pen or a syringe.

The dispensing of the substance occurs by simply exerting slight manual pressure on the tank or on the dispenser tip. There are no external parts to be broken and therefore there is no risk for the user of hurting him/herself.

The device may, advantageously, be produced by moulding, therefore at extremely limited production and assembly costs.

Thanks to these characteristics, the dispenser device proposed may be used in innumerable applications where there is a need to distribute a substance in a controlled manner. From a functional point of view, the various uses can be obtained merely by choosing the appropriate porosity of the dispenser tip.

## Claims

1. Dispenser device of a substance, comprising:
- a container-body (210) of an elongated shape extending around a longitudinal axis X and extending between two open extremities (214, 214') ;
- a tank (212) in the shape of a capsule for containing the substance to be dispensed, the tank having a proximal extremity (212') protruding from one extremity (214) of the container-body and a distal extremity fitted with a cap (216) facing the inside of said container-body and hermetically closing the tank (212);
- a perforation tip (218) attached to the inside of the container-body (210); and
- a dispenser tip (220) associated to the open extremity (214') of the container-body opposite the capsule and able to channel the substance coming out of the tank in a calibrated manner, the capsule being axially movable so as to cause breakage of the cap (216) by the perforation tip (218) by means of a manual pressure exerted on the proximal extremity (212'),
wherein the perforation tip (218) is made in one piece with the container-body (210) and comprises an attachment portion (218') for the connection to the container-body and a perforation head (218") which extends axially from said attachment portion (218') towards the cap (216) of the tank (212), wherein the perforation head (218") has a radial extension substantially equal to the internal cross section of the cap (216), and wherein the perforation tip (218) is blade-shaped in such a way to minimise contact with the fluid coming out of the cap (216).

2. Device according to claim 1, wherein said dispenser tip (220) is made from porous material.

3. Device according to claim 1, wherein said dispenser tip (220) is made from fabric.

4. Device according to claim 2, wherein said dispenser tip (220) is made from sintered material.

5. Device according to claim 2, wherein said dispenser tip (220) is made from spongy material.

6. Device according to anyone of the previous claims, wherein said attachment portion (218') is formed by a plurality of radial arms angularly equidistant to each other.

7. Device according to any of the previous claims, wherein the perforation head (218") comprises a plurality of primary perforation blades (219) radially equidistant to each other and converging axially towards the cap so as to form the apex of the perforation head, and a plurality of secondary perforation blades (219') radially equidistant to each other and alternated with the primary perforation blades (219), said secondary perforation blades (219') being axially convergent towards the cap (216) and terminating in a position rearward of the apex of the perforation head (218").

8. Device according to any of the previous claims, wherein the tank is inserted in the container-body (210) with a shaped coupling.

9. Device according to any of the previous claims, wherein the tank (212) is able to slide with calibrated interference along the inner walls of the container-body (210).

10. Device according to any of the previous claims, wherein, in the outer lateral surface of the tank (212) and/or in the inner surface of the container-body (210), means of transversal interference (222) between the tank (212) and container-body (210) are provided so as to define the positioning of the tank in relation to the container-body when the tank is rearward of the perforation tip.

11. Device according to any of the previous claims, wherein the dispenser tip (220) comprises a shank (220") at least partially accommodated in a respective portion of extremity of the container-body and a head (220') protruding from said portion of extremity.

12. Device according to claim 11, wherein on the inner lateral surface of the portion of the container-body holding the dispenser tip (220) there are a plurality of anchorage teeth (221) able to interact with the outer lateral surface of the shaft (220") of said dispenser tip to retain it axially.

13. Device according to claim 11 or 12, wherein the head (220') has a greater transversal section than the shaft (220") so as to abut against the rim defining the aperture of the container-body.

## Patentansprüche

1. Abgabevorrichtung für eine Substanz, die umfasst:
- einen Behälterkörper (210) mit einer länglichen Form, die sich um eine Längsachse X erstreckt und sich zwischen zwei offenen äußersten Enden (214, 214') erstreckt;
- einen Tank (212) in der Form einer Kapsel zum Aufnahmen der Substanz, die abgegeben werden soll, wobei der Tank ein proximales äußerstes Ende (212`), das von einem äußersten Ende (214) des Behälterkörpers vorsteht, und ein distales äußerstes Ende hat, das mit einer Kappe (216) ausgerüstet ist, die dem Inneren des Behälterkörpers zugewandt ist und den Tank (212) hermetisch verschließt;
- eine Perforierungsspitze (218), die an dem Inneren des Behälterkörpers (210) angebracht ist; und
- eine Abgabevorrichtungsspitze (220), die mit dem offenen äußersten Ende (214') des Behälterkörpers entgegengesetzt zu der Kapsel verbunden ist und fähig ist, die aus dem Tank kommende Substanz in einer kalibrierten Weise zu kanalisieren, wobei die Kapsel axial beweglich ist, um durch die Perforierungsspitze (218) mit Hilfe eines manuellen Drucks, der auf das proximale äußerste Ende (212') angewendet wird, den Bruch der Kappe (216) zu bewirken,
wobei die Perforierungsspitze (218) in einem Stück mit dem Behälterkörper (210) gefertigt ist und einen Befestigungsabschnitt (218') für den Anschluss an den Behälterkörper und einen Perforierungskopf (218") umfasst, der sich axial von dem ersten Befestigungsabschnitt (218') in Richtung der Kappe (216) des Tanks (212) erstreckt, wobei der Perforierungskopf (218") eine radiale Ausdehnung hat, die im Wesentlichen gleich dem inneren Querschnitt der Kappe (216) ist, und wobei die Perforierungsspitze (218) in einer derartigen Weise klingenförmig ist, dass der Kontakt mit dem aus der Kappe (216) kommenden Fluid minimiert wird.

2. Vorrichtung nach Anspruch 1, wobei die Abgabevorrichtungsspitze (220) aus porösem Material gefertigt ist.

3. Vorrichtung nach Anspruch 1, wobei die Abgabevorrichtungsspitze (220) aus Stoff bzw. Gewebe gefertigt ist.

4. Vorrichtung nach Anspruch 2, wobei die Abgabevorrichtungsspitze (220) aus gesintertem Material gefertigt ist.

5. Vorrichtung nach Anspruch 2, wobei die Abgabevorrichtungsspitze (220) aus schwammigem Material gefertigt ist.

6. Vorrichtung nach einem der vorhergehenden Ansprüche, wobei der Befestigungsabschnitt (218') durch eine Vielzahl radialer Arme ausgebildet ist, die winkelig gleichmäßig voneinander beabstandet sind.

7. Vorrichtung nach einem der vorhergehenden Ansprüche, wobei der Perforierungskopf (218") eine Vielzahl von primären Perforierungsklingen (219), die radial gleichmäßig voneinander beabstandet sind und axial in Richtung der Kappe konvergieren, um den Höhepunkt des Perforierungskopfs zu bilden, und eine Vielzahl von sekundären Perforierungsklingen (219') umfasst, die radial gleichmäßig voneinander beabstandet sind und sich mit den primären Perforierungsklingen (219) abwechseln, wobei die sekundären Perforierungsklingen (219') in Richtung der Kappe (216) axial konvergieren und an einer Position hinter dem Höhepunkt des Perforierungskopfs (218") enden.

8. Vorrichtung nach einem der vorhergehenden Ansprüche, wobei der Tank mit einer Formkupplung in den Behälterkörper (210) eingesetzt ist

9. Vorrichtung nach einem der vorhergehenden Ansprüche, wobei der Tank (212) fähig ist, mit einem kalibrierten Eingriff entlang der Innenwände des Behälterkörpers (210) zu gleiten.

10. Vorrichtung nach einem der vorhergehenden Ansprüche, wobei in der äußeren seitlichen Oberfläche des Tanks (212) und/oder in der Innenoberfläche des Behälterkörpers (210) Mittel zum Eingreifen (222) in Querrichtung zwischen dem Tank (212) und dem Behälterkörper (210) bereitgestellt sind, um die Positionierung des Tanks in Relation zu dem Behälterkörper zu definieren, wenn der Tank hinter der Perforierungsspitze ist.

11. Vorrichtung nach einem der vorhergehenden Ansprüche, wobei die Abgabevorrichtungsspitze (22) einen Kolben (220"), der wenigstens teilweise in einem jeweiligen Abschnitt des äußersten Endes des Behälterkörpers aufgenommen ist, und einen aus diesem Abschnitt des äußersten Endes vorstehenden Kopf (220') umfasst.

12. Vorrichtung nach Anspruch 11, wobei es auf der inneren seitlichen Oberfläche des Abschnitts des Behälterkörpers, welche die Abgabevorrichtungsspitze (220) hält, eine Vielzahl von Verankerungszähnen (221) gibt, die fähig sind, mit der äußeren seitlichen Oberfläche des Schafts (220") der Abgabevorrichtungsspitze zusammenzuwirken, um sie axial fest zu halten.

13. Vorrichtung nach Anspruch 11 oder 12, wobei der Kopf (220') einen größeren Querschnitt als der Schaft (220") hat, so dass er gegen den Rand anliegt, der die Öffnung des Behälterkörpers begrenzt bzw. definiert.

## Revendications

1. Dispositif de distribution d'une substance comprenant:
- un corps de récipient (210) d'une forme allongée s'étendant autour d'un axe longitudinal X et s'étendant entre deux extrémités ouvertes (214, 214');
- un réservoir (212) ayant la forme d'une capsule pour contenir la substance à distribuer, le réservoir ayant une extrémité proximale (212') faisant saillie de la première extrémité (214) du corps de récipient et une extrémité distale montée avec un capuchon (216) faisant face à l'intérieur du corps de récipient et fermant hermétiquement le réservoir (212);
- une pointe de perforation (218) fixée à l'intérieur du corps de récipient (210); et
- une pointe de distribution (220) fixée à l'extrémité ouverte (214') du corps de récipient opposée à la capsule et pouvant acheminer la substance sortant du réservoir d'une manière calibrée, la capsule étant axialement mobile afin de provoquer la rupture du capuchon (216) par la pointe de perforation (218) au moyen d'une pression manuelle exercée sur l'extrémité proximale (212'),
dans lequel la pointe de perforation (218) est réalisée d'une seule pièce avec le corps de récipient (210) et comprend une partie de fixation (218') pour le raccordement au corps de récipient et une tête de perforation (218") qui s'étend axialement à partir de ladite partie de fixation (218') vers le capuchon (216) du réservoir (212), dans lequel la tête de perforation (218'') a une extension radiale sensiblement égale à la section transversale interne du capuchon (216), et dans lequel la pointe de perforation (218) est en forme de lame afin de minimiser le contact avec le fluide sortant du capuchon (216).

2. Dispositif selon la revendication 1, dans lequel ladite pointe de distribution (220) est réalisée avec un matériau poreux.

3. Dispositif selon la revendication 1, dans lequel ladite pointe de distribution (220) est réalisée à partir d'un tissu.

4. Dispositif selon la revendication 2, dans lequel ladite pointe de distribution (220) est réalisée à partir d'un matériau fritté.

5. Dispositif selon la revendication 2, dans lequel ladite pointe de distribution (220) est réalisée à partir d'un matériau spongieux.

6. Dispositif selon l'une quelconque des revendications précédentes, dans lequel ladite partie de fixation (218') est formée par une pluralité de bras radiaux angulairement équidistants les uns des autres.

7. Dispositif selon l'une quelconque des revendications précédentes, dans lequel la tête de perforation (218") comprend une pluralité de lames de perforation principales (219) radialement équidistantes les unes des autres et convergeant axialement vers le capuchon afin de former le sommet de la tête de perforation, et une pluralité de lames de perforation secondaires (219') radialement équidistantes les unes des autres et alternées avec les lames de perforation principales (219), lesdites lames de perforation secondaires (219') étant axialement convergentes vers le capuchon (216) et se terminant par une position vers l'arrière du sommet de la tête de perforation (218").

8. Dispositif selon l'une quelconque des revendications précédentes, dans lequel le réservoir est inséré dans le corps de récipient (210) avec un couplage formé.

9. Dispositif selon l'une quelconque des revendications précédentes, dans lequel le réservoir (212) peut coulisser avec l'interférence calibrée le long des parois internes du corps de récipient (210).

10. Dispositif selon l'une quelconque des revendications précédentes, dans lequel, dans la surface latérale externe du réservoir (212) et/ou dans la surface interne du corps de récipient (210), on prévoit des moyens d'interférence transversale (222) entre le réservoir (212) et le corps de récipient (210) afin de définir le positionnement du réservoir par rapport au corps de récipient lorsque le réservoir est vers l'arrière de la pointe de perforation.

11. Dispositif selon l'une quelconque des revendications précédentes, dans lequel la pointe de distribution (220) comprend une tige (220") logée au moins partiellement dans une partie d'extrémité respective du corps de récipient et une tête (220') faisant saillie de ladite partie d'extrémité.

12. Dispositif selon la revendication 11, dans lequel, sur la surface latérale interne de la partie du corps de récipient maintenant la pointe de distribution (220), il y a une pluralité de dents d'ancrage (221) pouvant interagir avec la surface latérale externe de la tige (220'') de ladite pointe de distribution pour la retenir axialement.

13. Dispositif selon la revendication 11 ou 12, dans lequel la tête (220') a une plus grande section transversale que la tige (220 ") afin de venir en butée contre le bord définissant l'ouverture du corps de récipient.
